(19) Europäisches Patentamt
European Patent Office
Office européen des brevets

(11) Veröffentlichungsnummer: **0 189 800**
**A2**

(12) # EUROPÄISCHE PATENTANMELDUNG

(21) Anmeldenummer: 86100631.0

(22) Anmeldetag: 18.01.86

(51) Int. Cl.[4]: C 07 C 161/02
C 07 D 251/38, C 07 D 231/12
A 01 N 43/56, A 01 N 43/64
A 01 N 47/28

(30) Priorität: 30.01.85 DE 3502926

(43) Veröffentlichungstag der Anmeldung:
06.08.86 Patentblatt 86/32

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Brandes, Wilhelm, Dr.
Eichendorffstrasse 3
D-5653 Leichlingen(DE)

(72) Erfinder: Bunnenberg, Rolf, Dr.
Neuenkamper Weg 12
D-5653 Leichlingen(DE)

(72) Erfinder: Hänssler, Gerd, Dr.
Am Arenzberg 58a
D-5090 Leverkusen 3(DE)

(72) Erfinder: Reinecke, Paul, Dr.
Lessingstrasse 11
D-5090 Leverkusen 3(DE)

(72) Erfinder: Wedemeyer, Karlfried, Dr.
Bilharzstrasse 11
D-5000 Koeln 80(DE)

(54) **Thiocyanatomethylthio-Gruppen enthaltende Verbindungen.**

(57) Thiocyanatomethylthio-Gruppen enthaltende Verbindungen der allgemeinen Formel (I)

$$A-CO-N=\underset{}{\overset{B}{\overset{|}{C}}}-S-CH_2-SCN \qquad (I)$$

in welcher

A und B die im Text angegebene Bedeutung haben sowie ihre Verwendung als Schädlingsbekämpfungsmittel, vor allem als Fungizide.

Die neuen Thiocyanatomethylthio-Gruppen enthaltenden Verbindungen können hergestellt werden, wenn man Verbindungen der allgemeinen Formel (II)

$$A-CO-NH-CS-B \qquad (II)$$

in welcher

A und B die im Text angegebenen Bedeutungen haben, mit geeigneten Halogenmethylthiocyanaten umsetzt.

EP 0 189 800 A2

BAYER AKTIENGESELLSCHAFT 5090 Leverkusen, Bayerwerk
Konzernverwaltung RP Bas/by-c
Patentabteilung Ia

# Thiocyanatomethylthio-Gruppen enthaltende Verbindungen

Die vorliegende Erfindung betrifft neue Thiocyanatomethylthio-Gruppen enthaltende Verbindungen, ein Verfahren zu ihrer Herstellung und ihre Verwendung als Schädlingsbekämpfungsmittel, vor allem als Fungizide.

Es ist bereits bekannt, daß Thiocyansäurederivate, wie z.B. das Methylenbisrhodanid oder das 2,4-Dinitrophenylthiocyanat, fungizid wirken (vgl. z.B. R. Wegler, Chemie der Pflanzenschutz- und Schädlingsbekämpfungsmittel, Bd. 2, Seite 69, Springer Verlag Berlin-Heidelberg-New York (1970)).

Weiterhin sind Thiocyanatomethylthio-Verbindungen bekannt, wie z.B. das 2-Thiocyanatomethylthio-benzo-1,3-thiazol (vgl. US 3.520.976), das Thiocyanatomethylthio-ethoxymethylidenanilin (vgl. JP 58-083669 und Alkyl- und Arylcarbonsäure-S-rhodanomethylester (vgl. JP 73-08429 und EP 001623). Auch diese Verbindungen zeigen gute fungizide Eigenschaften.

Weiterhin ist bekannt, daß schwefelhaltige organische Verbindungen, wie beispielsweise das Zinkethylen-1,2-bis-(dithiocarbamat), das N-Dichlorfluormethylthio-N-phenyl-N',N'-dimethyl-sulfamid und das N-Trichlormethylthio-phthalimid, fungizide Eigenschaften besitzen (vgl. z.B. R. Wegler, "Chemie der Pflanzenschutz- und Schädlingsbekämpfungsmittel", Springer-Verlag Berlin-Heidelberg-New York, 1970, Bd. 2, S. 65 ff, S. 95 und S. 109). Bei all diesen Verbindungen kann unter bestimmten Umständen, z.B. bei niedrigen Anwendungskonzentrationen, die Wirkung nicht immer voll befriedigend sein.

Es wurden neue Thiocyanatomethylthio-Gruppen enthaltende Verbindungen der allgemeinen Formel (I)

$$A\text{-}CO\text{-}N\text{=}\overset{B}{\overset{|}{C}}\text{-}S\text{-}CH_2\text{-}SCN \qquad (I)$$

in welcher

A für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^1X$ steht, wobei

X für Sauerstoff, Schwefel oder $-NR^3$ steht,

$R^1$ für gegebenenfalls ein- bis mehrfach, gleich oder verschieden durch Halogen, Nitro, Rhodan,

Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl oder für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, außerdem für Wasserstoff stehen kann, wenn X für $-NR^3$ steht,

$R^3$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht,

B für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^2Y$ steht, wobei

Y für Sauerstoff, Schwefel oder $-NR^4$ steht,

$R^2$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl und für 5- oder 6-gliedrige Heterocyclen, die 1 bis 4 gleiche oder verschiedene Heteroatome enthalten und gegebenenfalls ein- oder mehrfach, gleich oder verschieden durch Halogen, Alkyl und Halogenalkyl substituiert sind und gegebenenfalls ankondensierte Ringe

enthalten, steht, außerdem für Wasserstoff stehen kann, wenn Y für $-NR^4$ steht,

$R^4$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht, oder

A und B gemeinsam die Gruppe $-NR^5-CZ-NR^6$ bedeuten, wobei

$R^5$ und $R^6$ unabhängig voneinander für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder

Aryl oder für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl, und Halogenalkyl substituiert sind und gegebenenfallls ankondensierte Ringe enthalten, stehen

und

Z für Sauerstoff oder Schwefel steht,

gefunden.

Für den Fall, daß A und B gemeinsam die Gruppe $-NR^5-CZ-R^6$ bilden, entsteht das heterocyclische System der allgemeinen Formel (IA)

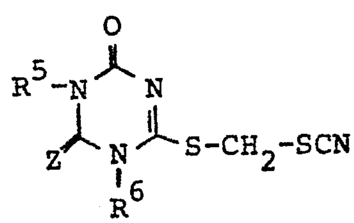

(IA)

worin

$R^5$, $R^6$ und Z die oben angegebene Bedeutung haben.

Man erhält die Thiocyanatomethylthio-Gruppen enthaltenden Verbindungen der Formel (I)

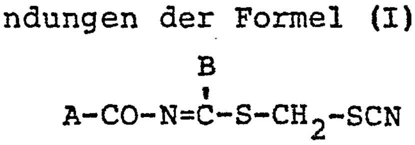

(I)

in welcher

A für 5- oder 6-gliedrige Heterocyclen, die 1 bis 4 gleiche oder verschiedene Heteroatome enthalten und gegebenenfalls ein- bis mehrfach gleich oder verschieden durch Halogen, Alkyl und Halogenalkyl substituiert sind und gegebenenfalls ankondensierte Ringe enthalten, steht, oder für den Rest $R^1X$ steht, wobei

X für Sauerstoff, Schwefel oder $-NR^3$ steht,

$R^1$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl oder für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert

sind und gegebenenfalls ankondensierte Ringe enthalten, steht, außerdem für Wasserstoff stehen kann, wenn X für $-NR^3$ steht,

$R^3$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht

B für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^2Y$ steht, wobei

Y für Sauerstoff, Schwefel oder $-NR^4$ steht,

$R^2$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für

gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl und für 5- oder 6-gliedrige Heterocyclen, die 1 bis 4 gleiche oder verschiedene Heteroatome enthalten und gegebenenfalls ein- oder mehrfach, gleich oder verschieden durch Halogen, Alkyl und Halogenalkyl substituiert sind und gegebenenfalls ankondensierte Ringe enthalten, steht, außerdem für Wasserstoff stehen kann, wenn Y für $-NR^4$ steht,

$R^4$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht, oder

A und B gemeinsam die Gruppe $-NR^5-CZ-NR^6$ bedeuten, wobei

$R^5$ und $R^6$ unabhängig voneinander für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl,

für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl oder für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl, und Halogenalkyl substituiert sind und gegebenenfallls ankondensierte Ringe enthalten, stehen

und

Z für Sauerstoff oder Schwefel steht,

wenn man Verbindungen der allgemeinen Formel (II)

A-CO-NH-CS-B (II)

in welcher

A und B die oben angegebene Bedeutung haben,

mit Halogenmethylthiocyanaten der Formel (III)

$$Hal-CH_2-SCN \qquad (III)$$

in welcher

Hal für Chlor, Brom oder Iod steht,

in Gegenwart von Verdünnungsmitteln und gegebenenfalls in Gegenwart einer Base bei Temperaturen zwischen -50°C und +120°C umsetzt.

Die neuen Thiocyanatomethylthio-Gruppen enthaltenden Verbindungen der Formel (I) weisen starke Wirkungen gegen Schädlinge, besonders gegen pilzliche Erreger auf.

Überraschenderweise zeigen die erfindungsgemäßen Thiocyanatomethylthio-Gruppen enthaltenden Verbindungen eine erheblich höhere Wirkung gegen Schädlinge, vor allem gegen pilzliche Erreger als die aus dem Stand der Technik bekannten Verbindungen gleicher Wirkungsrichtung.

Von den erfindungsgemäßen Thiocyanatomethylthio-Gruppen enthaltenden Verbindungen der Formel (I) sind bevorzugt diejenigen, in denen

A für gesättigte oder ungesättigte 5- oder 6-gliedrige Heterocyclen mit 1 bis 4 gleichen oder verschiedenen Hetero-Atomen, wie Sauerstoff, Stickstoff oder Schwefel, steht, die gegebenenfalls ein- bis fünffach, gleich oder verschieden durch Halogen, wie Fluor, Chlor, Brom, Iod, Alkyl mit 1 bis 4 Kohlenstoffatomen und Halogenalkyl mit 1 bis 3 Kohlenstoffatomen und 1 bis 5 gleichen oder verschiedenen Halogenatomen, wie Fluor, Chlor, Brom, Iod, substituiert sind und gegebenenfalls einen ankondensierten Benzolring enthalten, oder für den Rest $R^1X$ steht, wobei

X für Sauerstoff, Schwefel oder $-NR^3$ steht,

$R^1$ für gegebenenfalls ein- bis fünffach, gleich oder verschieden durch Halogen, wie Fluor, Chlor, Brom, Iod, durch Nitro, Rhodan, Cyan, Alkoxy mit 1 bis 4 Kohlenstoffatomen und Alkylthio mit 1 bis 4 Kohlenstoffatomen substituiertes Alkyl mit 1 bis 16 Kohlenstoffatomen, für Alkenyl oder Alkinyl mit jeweils 2 bis 6 Kohlenstoffatomen, für Halogenalkenyl oder Halogenalkinyl mit 2 bis 6 Kohlenstoffatomen und 1 bis 5 gleichen oder verschiedenen Halogenatomen, wie Fluor, Chlor, Brom, Iod, für jeweils gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Halogenalkyl mit 1 bis 4 Kohlenstoffatomen und

1 bis 5 gleichen oder verschiedenen Halogenatomen wie Fluor, Chlor, Brom und Iod, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Halogenatomen, wie Fluor, Chlor, Brom und Iod, substituiertes Cycloalkyl oder Cycloalkenyl mit 5 bis 7 Kohlenstoffatomen, für gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Halogenalkyl mit 1 bis 4 Kohlenstoffatomen und 1 bis 5 gleichen oder verschiedenen Halogenatomen, wie Fluor, Chlor, Brom und Iod, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Halogenatomen, wie Fluor, Chlor, Brom und Iod, jeweils im Arylteil substituiertes Aralkyl oder Aryl mit jeweils 6 bis 10 Kohlenstoffatomen im Aryl und gegebenenfalls 1 bis 4 Kohlenstoffatomen im Alkylteil, und für 5- oder 6-gliedrige Heterocyclen mit 1 bis 4 gleichen oder verschiedenen Heteroatomen, wie Sauerstoff, Schwefel oder Stickstoff, die gleich oder verschieden, ein- bis vierfach durch Halogen, wie Fluor, Chlor,

Brom und Iod, Alkyl mit 1 bis 4 Kohlenstoffatomen und Halogenalkyl mit 1 bis 4 Kohlenstoffatomen und 1 bis 5 gleichen oder verschiedenen Halogenatomen, wie Fluor, Chlor, Brom und Iod substituiert sein und einen ankondensierten Benzolring enthalten können, außerdem für Wasserstoff stehen kann, wenn X für $-NR^3$ steht,

$R^3$ für Wasserstoff, für Alkyl mit 1 bis 6 Kohlenstoffatomen, für gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und jeweils 1 bis 5 Halogenatomen, wie Fluor, Chlor, Brom und Iod, jeweils substituiertes Aralkyl und Aryl mit 6 bis 10 Kohlenstoffatomen je Aryl und 1 bis 4 Kohlenstoffatomen im Alkylteil steht,

B für gesättigte oder ungesättigte 5- oder 6-gliedrige Heterocyclen mit 1 bis 4 gleichen oder verschiedenen Heteroatomen wie Sauerstoff, Stickstoff oder Schwefel steht,
die gegebenenfalls ein- bis fünffach, gleich oder verschieden durch Halogen, wie Fluor, Chlor, Brom,

Iod, Alkyl mit 1 bis 4 Kohlenstoffatomen und Halogenalkyl mit 1 bis 3 Kohlenstoffatomen und 1 bis 5 gleichen oder verschiedenen Halogenatomen, wie Fluor, Chlor, Brom, Iod, substituiert sind und gegebenenfalls einen ankondensoerten Benzolring enthalten, steht, oder für den Rest $R^2$-Y steht, wobei

Y für Sauerstoff, Schwefel oder $-NR^4$ steht,

$R^2$ für gegebenenfalls ein- bis fünffach, gleich oder verschieden durch Halogen, wie Fluor, Chlor, Brom, Iod, durch Nitro, Rhodan, Cyan, Alkoxy mit 1 bis 4 Kohlenstoffatomen und Alkylthio mit 1 bis 4 Kohlenstoffatomen substituiertes Alkyl mit 1 bis 16 Kohlenstoffatomen, für Alkenyl oder Alkinyl mit jeweils 2 bis 6 Kohlenstoffatomen, für Halogenalkenyl oder Halogenalkinyl mit 2 bis 6 Kohlenstoffatomen und 1 bis 5 gleichen oder verschiedenen Halogenatomen, wie Fluor, Chlor, Brom, Iod, für jeweils gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Halogenalkyl mit 1 bis 4 Kohlenstoffatomen und 1 bis 5 gleichen oder verschiedenen Halogenatomen wie Fluor, Chlor, Brom und Iod, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen

Halogenatomen, wie Fluor, Chlor, Brom und Iod, jeweils substituiertes Cycloalkyl oder Cycloalkenyl mit 5 bis 7 Kohlenstoffatomen, für gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Halogenalkyl mit 1 bis 4 Kohlenstoffatomen und 1 bis 5 gleichen oder verschiedenen Halogenatomen, wie Fluor, Chlor, Brom und Iod, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Halogenatomen, wie Fluor, Chlor, Brom und Iod, jeweils im Arylteil substituiertes Aralkyl oder Aryl mit jeweils 6 bis 10 Kohlenstoffatomen im Aryl und gegebenenfalls 1 bis 4 Kohlenstoffatomen im Alkylteil, und für 5- oder 6-gliedrige Heterocyclen mit 1 bis 4 gleichen oder verschiedenen Heteroatome wie Sauerstoff, Schwefel oder Stickstoff, die gleich oder verschieden, ein- bis vierfach durch Halogen, wie Fluor, Chlor, Brom und Iod, Alkyl mit 1 bis 4 Kohlenstoffatomen und Halogenalkyl mit 1 bis 4 Kohlenstoffatomen und 1 bis 5 gleichen oder verschiedenen Halogenatomen, wie Fluor, Chlor, Brom und Iod, substituiert sein können und einen ankondensierten Benzolring enthalten können, außerdem für Wasserstoff stehen kann, wenn Y für $-NR^4$ steht,

0189800

$R^4$ für Wasserstoff, für Alkyl mit 1 bis 6 Kohlenstoffatomen, für gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und jeweils 1 bis 5 Halogenatomen, wie Fluor, Chlor, Brom und Iod, jeweils substituiertes Aralkyl und Aryl mit 6 bis 10 Kohlenstoffatomen je Arylrest und 1 bis 4 Kohlenstoffatome im Alkylteil steht, oder

A und B gemeinsam die Gruppe $-NR^5-CZ-NR^6$ bedeuten, wobei

$R^5$ und $R^6$ unabhängig voneinander für gegebenenfalls ein- bis fünffach, gleich oder verschieden durch Halogen, wie Fluor, Chlor, Brom und Iod, Nitro, Rhodan, Cyan, Alkoxy oder Alkylthio mit jeweils 1 bis 4 Kohlenstoffatomen substituiertes Alkyl mit 1 bis 6 Kohlenstoffatomen für Alkenyl oder Alkinyl mit jeweils bis zu 6 Kohlenstoffatomen, für Halogenalkenyl oder Halogenalkinyl mit bis zu 4 Kohlenstoffatomen und 1 bis 5 gleichen oder verschiedenen Halogenatomen, wie Fluor, Chlor, Brom und Iod, für jeweils gegebenenfalls ein- bis fünffach, gleich oder verschieden durch Alkyl mit

1 bis 4 Kohlenstoffatomen, Halogen, wie Fluor, Chlor, Brom und Iod, Nitro, Rhodan, Cyan, Halogenalkyl mit 1 bis 4 Kohlenstoffatomen und 1 bis 5 gleichen oder verschiedenen Halogenatomen wie Fluor, Chlor, Brom und Iod, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Halogenatomen, wie Fluor, Chlor, Brom und Iod, jeweils substituiertes Cycloalkyl oder Cycloalkenyl mit 5 bis 7 Kohlenstoffatomen, für gegebenenfalls ein- bis fünffach, gleich oder verschieden durch Alkyl mit 1 bis 4 Kohlenstoffatomen, Halogen, wie Fluor, Chlor, Brom und Iod, Nitro, Rhodan, Cyan, Halogenalkyl mit 1 bis 4 Kohlenstoffatomen und 1 bis 5 gleichen oder verschiedenen Halogenatomen, wie Fluor, Chlor, Brom und Iod, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Halogenatomen, wie Fluor, Chlor, Brom und Iod, jeweils im Arylteil substituiertes Aralkyl oder Aryl mit jeweils 6 bis 10 Kohlenstoffatomen im Aryl und gegebenenfalls 1 bis 4 Kohlenstoffatomen im Alkylteil, und für 5- oder 6-gliedrige Heterocyclen mit 1 bis 4 gleichen oder verschiedenen Heteroatomen wie Sauerstoff, Schwefel oder Stickstoff, die gleich oder verschieden, ein- bis vierfach durch Halogen, wie Fluor, Chlor, Brom und Iod,

Alkyl mit 1 bis 4 Kohlenstoffatomen und Halogenalkyl mit 1 bis 4 Kohlenstoffatomen und 1 bis 5 gleichen oder verschiedenen Halogenatomen, wie Fluor, Chlor, Brom und Iod, substituiert sein können und einen ankonsierten Benzolring enthalten können, stehen und

Z für Sauerstoff oder Schwefel steht.

Ganz besonders bevorzugt sind Verbindungen der Formel (I), in denen

A für Piperidin, Piperazin, Pyrrolidin, Imidazolidin, Imidazol, Benzimidazolin, Pyrazolin, Carbazolin, Morpholin, Thiomorpholin oder für $R^1X$ steht, wobei

X für Sauerstoff oder $-NR^3$ steht,

$R^1$ für gegebenenfalls ein- bis fünffach, gleich oder verschieden durch Fluor und Chlor, Methoxy, Ethoxy, Methylthio oder Ethylthio substituiertes Alkyl mit 1 bis 6 Kohlenstoffatomen steht, außerdem für Wasserstoff steht, wenn X für $-NR^3$ steht,

$R^3$ für Wasserstoff, für Alkyl mit 1 bis 6 Kohlenstoffatomen, für gegebenenfalls ein- bis dreifach, gleich oder verschieden durch Fluor, Chlor, Methyl, Ethyl, n- und iso-Propyl, Trifluormethyl, Trichlormethyl, Trifluormethoxy,

Trichlormethoxy, Trifluormethylthio, Dichlorfluormethyl, Dichlorfluormethoxy jeweils substituiertes Phenyl, Benzyl oder Phenethyl steht,

B für Piperidin, Piperazin, Pyrrolidin, Imidazolin, Imidazolidin, Benzimidazolin, Pyrazolin, Carbazolin, Morpholin oder Thiomorpholin steht, weiterhin für den Rest $R^2Y$ steht, wobei

Y für Sauerstoff, Schwefel oder $-NR^4$ steht,

$R^2$ für gegebenenfalls ein- bis vierfach durch Fluor, Chlor, Methoxy, Ethoxy, Methylthio und Ethylthio substituiertes Alkyl mit 1 bis 14 Kohlenstoffatomen, für Benzyl, für gegebenenfalls ein- bis dreifach, gleich oder verschieden durch Fluor, Chlor, Methyl, Ethyl, Methoxy, Ethoxy, Methylthio, Ethylthio, Trifluormethyl, Trichlormethyl, Dichlorfluormethyl, Dichlorfluormethyl oder Tetrachlorethyl substituiertes Phenyl steht, außerdem für Wasserstoff stehen kann, wenn Y für $-NR^4$ steht,

$R^4$ für Wasserstoff, für Alkyl mit 1 bis 4 Kohlenstoffatomen oder für gegebenenfalls ein- bis vierfach durch Fluor, Chlor, Methoxy, Ethoxy, Methylthio und Ethylthio substituiertes Alkyl mit 1 bis 14 Kohlenstoffatomen, für Benzyl, für gegebenenfalls ein- bis dreifach, gleich oder verschieden durch Fluor, Chlor, Methyl, Ethyl, Methoxy, Ethoxy, Methylthio, Ethylthio, Trifluormethyl, Trichlormethyl, Dichlorfluormethyl, Dichlorfluormethoxy oder Tetrachlorethyl substituiertes Phenyl steht, oder

A und B gemeinsam für die Gruppe $-NR^5-CZ-NR^6$ stehen, wobei

$R^5$ und $R^6$ unabhängig voneinander für gegebenenfalls durch Fluor, Chlor, Methoxy, Ethoxy, Methylthio oder Ethylthio substituiertes Alkyl mit 1 bis 4 Kohlenstoffatomen oder für Phenyl stehen und

Z für Sauerstoff steht.

Verwendet man beispielsweise 4-(3,5-Dichlorphenyl)-thioallophansäure-O-methylester und Chlormethylrhodanid als Ausgangsstoffe, so kann der Reaktionsablauf durch das folgende Formelschema wiedergegeben werden:

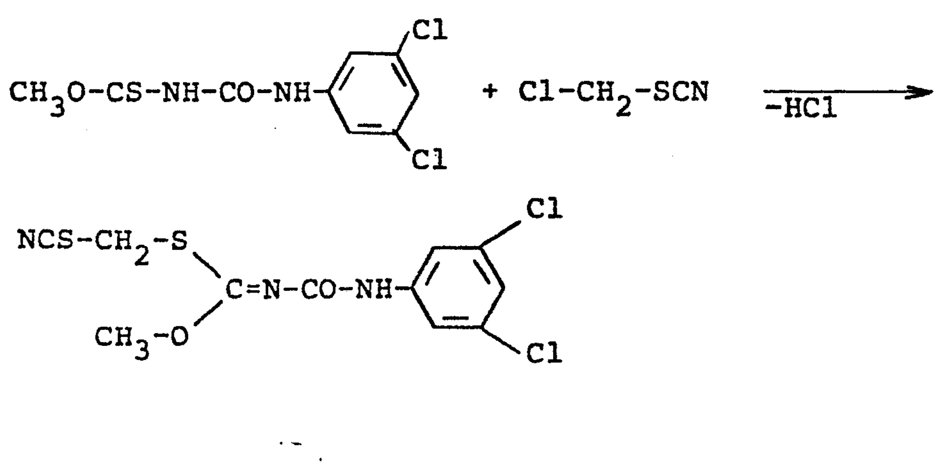

Die bei der Durchführung des erfindungsgemäßen Verfahrens verwendeten Ausgangsstoffe sind durch die Formeln (II) und (III) allgemein definiert.

Unter die allgemeine Formel (II) fallen beispielsweise die folgenden Untergruppen: Thiobiurete (mit A = $NR^1R^3$ und B = $NR^2R^4$), Thioallophansäure-O-ester (mit A = $NR^1R^3$ und B = $OR^2$ oder A = $OR^1$ und B = $NR^2R^4$), Dithioallophansäureester (mit A = $NR^1R^3$ und B = $SR^2$ oder A = $SR^1$ und B = $NR^2R^4$), Oxycarbonylthiocarbamidsäure-O-ester (mit A = $OR^1$ und B = $OR^2$), Thiocarbonylthiocarbamidsäure-O-ester (mit A = $SR^1$ und B = $OR^2$), Oxycarbonyldithiocarbamidsäureester (mit A = $OR^1$ und B = $SR^2$), Thiocarboxydithiocarbamidsäureester (mit A = $SR^1$ und B = $SR^2$), 2-Thioxo-1,3,5-triazin-4,6-dion (mit A und B = $-NR^5-CO-NR^6-$) und 2,4-Dithioxo-1,3,5-triazin-6-on (mit A und B = $-NR^5-CS-NR^6-$).

Viele Einzelverbindungen dieser Untergruppen sind bekannt und können nach literaturbekannten Analogieverfahren herstellt werden: (vgl. z.B. DE-OS 3 010 237, DE-OS 3 010 238, J. Goerdeler, K. Jones, Chem. Ber. 99, 3572 (1966); J. Goerdeler, O. Wobig, Liebigs Ann. Chem. 731, 120 (1970); A.E. Dixon, J. Chem. Soc. 75, 388 (1899); R.E. Doran, J. Chem. Soc. 79, 906 (1901) und R. Esmail, F. Kurzer, Synthesis 1975, 301).

Die weiterhin als Ausgangsstoffe benötigten Halogenmethylrhodanide der Formel (II) sind ebenfalls bekannt

(vgl. z.B. Bull. Sci. Pharmacol. 29, 425 (1922), EP 0.065.190, J 7.606.927).

Als Verdünnungsmittel kommen Wasser, organische Lösungsmittel sowie Gemische in Frage. Hierzu gehören vorzugsweise Alkohole, wie beispielsweise Methanol, Ethanol, Propanol, i-Propanol; Ketone, wie beispielsweise Aceton, Methylethylketon, Diethylketon; Ether, wie beispielsweise Diethylether, Dimethoxyethan, Tetrahydrofuran, Dioxan; Kohlenwasserstoffe, wie beispielsweise Hexan, Ligroinfraktionen, Benzol, Toluol, Xylol; halogenierte Kohlenwasserstoffe, wie beispielsweise Methylenchlorid, Chloroform, Tetrachlorkohlenstoff, Chlorbenzol und Dichlorbenzol und polare, aprotische Lösungsmittel, wie beispielsweise Dimethylsulfoxid, Dimethylacetamid, Dimethylformamid, N-Methylpyrrolidon. Wird Wasser als Lösungsmittel verwendet, kann ein Zusatz von Phasentransferkatalysatoren zweckmäßig sein. Besonders bevorzugt werden Alkohole, Ether und polare aprotische Lösungsmittel.

Als Basen können Alkali- oder Erdalkalioxide, -hydroxide, -carbonate, -hydrogencarbonate, -hydride oder -alkoholate sowie organische Verbindungen, wie beispielsweise Triethylamin, Tributylamin, Benzyldimethylamin, Dimethylanilin, Pyridin, Chinolin, DBN oder DBU verwendet werden. Die Reaktionstemperaturen können in einem größeren Bereich variiert werden. Im allgemeinen arbeitet man zwischen -50°C und +120°C, vorzugsweise bei +10°C bis +60°C.

Bei der Durchführung des erfindungsgemäßen Verfahrens setzt man auf 1 Mol der Verbindung der Formel (II) 1 bis 10 Mol, vorzugsweise 1,5 bis 5 Mol der Verbindung der Formel (III) ein. Zur Isolierung der Verbindungen der Formel (I) wird von ausgefallenen Salzen abfiltriert und das Verdünnungsmittel sowie überschüssiges Halogenmethylrhodanid im Vakuum abgedampft. Bei mit Wasser mischbaren Verdünnungsmitteln kann der Reaktionsansatz auf Eiswasser gegeben und das ausgeschiedene Produkt abgetrennt werden. Die erfindungsgemäßen Verbindungen der Formel (I) können gegebenenfalls durch Destillation, Extraktion oder Kristallisation gereinigt werden.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch als Schädlingsbekämpfungsmittel, vor allem als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Botrytis-Arten, wie beispielsweise Botrytis cinerea;
Plasmopara-Arten, wie beispielsweise Plasmopara viticola;
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder Ustilago avenae;
Septoria-Arten, wie beispielsweise Septoria nodorum;
Tilletia-Arten, wie beispielsweise Tilletia caries;
Pyricularia-Arten, wie beispielsweise Pyricularia oryzae;
Pellicularia-Arten, wie beispielsweise Pellicularia sasakii und
Pyrenophora-Arten, wie beispielsweise Pyrenophora teres.
(Konidienform: Drechslera, Syn: Helminthosporium);
Leptosphaeria-Arten, wie beispielsweise Leptosphaeria nodorum;
Cochliobolus-Arten, wie beispielsweise Cochliobolus sativus
(Konidienform: Drechslera, Syn: Helminthosporium) und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten, wie beispielsweise verursacht durch Cochliobolus

sativus oder Pyrenophora teres, gegen Rosterreger, gegen Mehltauerreger, zur Bekämpfung von Tomatenkrankheiten, wie beispielsweise gegen den Erreger der Tomatenbraunfäule (Phytophthora infestans) oder zur Bekämpfung von durch Venturia inaequalis verursachte Krankheiten am Apfel, außerdem zur Bekämpfung von Reiskrankheiten, verursacht z.B. durch den Erreger Pyricularia oryzae, eingesetzt werden. Außerdem zeigen die Verbindungen eine in vitro-Wirkung. Bei entsprechenden Konzentrationen und Anwendungen sind die Verbindungen auch akarizid wirksam und zeigen eine Wirksamkeit gegen Hygiene- und Vorratsschädlinge.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B.

Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

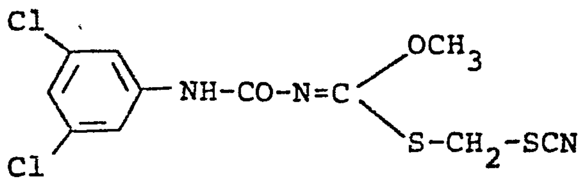

Zu 314 g (1,13 Mol) 4-(3,5-Dichlorphenyl)thioallophansäure-O-methylester in 2,5 l Dimethylformamid tropft man unter Eiskühlung die Lösung von 69 g (1,24 Mol) Kaliumhydroxid in 1,1 l Wasser. Man rührt 60 Minuten bei 22°C nach und tropft diese Lösung zu 182 g (1,69 Mol) Chlormethylrhodanid in 1,1 l Dimethylformamid. Es wird 3 h nachgerührt, das Reaktionsgemisch auf Eiswasser gekippt und der Niederschlag nach 60 Minuten abgesaugt und getrocknet. Man erhält 325 g (82 % der Theorie) N-(3,5-Dichlorphenyl)-N-(methoxy-thiocyanatomethylthio)-methylidenharnstoff als farbloses Pulver vom Schmelzpunkt 153 bis 154°C.

IR (KBr): NH = 3320 $cm^{-1}$, SCN 2160, CO 1670

$^{1}H$-NMR ($CDCl_3$): $CH_3$ = 4,06 ppm (s), $CH_2$ 4,40 (s), Aromat 7,06 und 7,48 (je s), NH 7,33 (s)

| | | | | | |
|---|---|---|---|---|---|
| $C_{11}H_9Cl_2N_3O_2S_2$ (350,1) | ber. | C 37,7 | H 2,6 | N 12,0 |
| | gef. | 37,7 | 2,7 | 11,7 |

Herstellung des Ausgangsmaterials:

Zu 288 g (2 Mol) Carbonyldisenföl in 1 l Tetrahydrofuran tropft man bei 0°C die Lösung von 64 g (2 Mol) Methanol in 1 l Tetrahydrofuran. Es wird 30 min nachgerührt und bei 22°C die Lösung von 324 g (2 Mol) 3,5-Dichloranilin und 202 g (2 Mol) Triethylamin in 2 l Tetrahydrofuran zugetropft. Nach 3 h wird das Lösungsmittel abgedampft, der Rückstand mit Wasser aufgenommen, rhodanidfrei gewaschen und getrocknet. Man erhält 518 g (93 % der Theorie) farblose Kristalle vom Schmelzpunkt 186 bis 187°C.

Beispiel 2

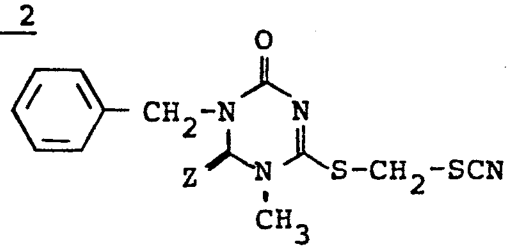

Zu 6,7 g (0,06 Mol) Kalium-tert.-butylat in 30 ml Dimethylformamid tropft man bei 22°C die Lösung von 12,5 g (0,05 Mol) 1-Methyl-5-benzyl-2-thioxo-1,3,5-triazin-4,6-dion in 50 ml Dimethylformamid. Es wird 2 h nachgerührt und diese Lösung zu 10,6 g (0,07 Mol) Brommethylrhodanid in 20 ml Dimethylformamid getropft. Nach 16 h Rühren bei 22°C wird vom Niederschlag abgesaugt, das Filtrat im Vakuum zur Trockne eingedampft, mit Methylenchlorid aufgenommen und mit Wasser gewaschen. Die organische Phase wird über Calciumchlorid getrocknet und das Lösungsmittel abdestilliert. Man erhält 15,0 g (94 % der Theorie) 1-Methyl-2-thiocyanatomethylthio-5-benzyl-1,3,5-triazin-4,6-dion als honigartiges Harz.

IR (Film): SCN = 2160 $cm^{-1}$, CO 1735 und 1675

$^1$H-NMR ($CDCl_3$): $CH_3$ = 3,4 ppm (s), $CH_2$ 4,6 und 5,0 (je s), Aromat um 7,3 (m)

<u>Darstellung des Ausgangsmaterials</u>:

Zu 14,4 g (0,1 Mol) Carbonyldisenföl in 100 ml Tetrahydrofuran tropft man bei 0°C die Lösung von 16,4 g (0,1 Mol) N-Methyl-N'-benzylharnstoff in 200 ml Tetrahydrofuran. Nach 60 min wird die Lösung von 10,1 g (0,1 Mol) Triethylamin in 100 ml Tetrahydorfuran zugetropft. Man läßt 90 min nachrühren, dampft im Vakuum zur Trockne ein, nimmt den Rückstand mit Eiswasser auf, filtriert und wäscht den Filterkuchen rhodanidfrei. Nach dem Trocknen erhält man 20,8 g (84 % der Theorie) farblose Kristalle vom Schmelzpunkt 190 bis 191°C.

$^1$H-NMR ($CDCl_3$): $CH_3$ = 3,63 ppm (s), $CH_2$ 5,00 (s), Aromat um 7,3 (m), NH 11,7 (breit)

| | | | | |
|---|---|---|---|---|
| $C_{11}H_{11}N_3O_2S$ (249,1) | ber.: | C 53,0 | H 4,4 | N 16,9 |
| | gef.: | 53,2 | 4,4 | 17,0 |

In entsprechender Weise wie Beispiele 1 und 2 werden die nachfolgenden Beispiele der allgemeinen Formel (I)

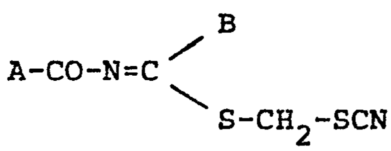

(I)

erhalten.

| Bsp. Nr. | A | B | Schmelzpunkt (°C) |
|---|---|---|---|
| 3 | $n-C_4H_9-NH-$ | $-O-C_2H_5$ | Öl |
| 4 | $(CH_3)_2N-$ | $-O-CH_2-C_6H_5$ | 48-52°C |
| 5 | 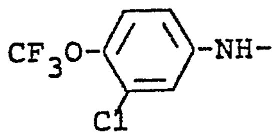 | $-O-C_2H_5$ | 110-113 |
| 6 | $(CH_3)_3CCH_2-NH-$ | $-O-C_5H_{11}$ | Öl |
| 7 | 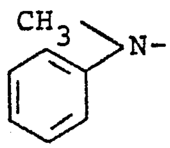 | $-O-C_{14}H_{29}$ | Öl |
| 8 | $(CH_3)_3CCH_2NH-$ | $-S-C_2H_5$ | 76-79 |
| 9 | 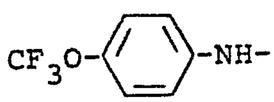 | $-S-C_2H_5$ | 91-93 |
| 10 | 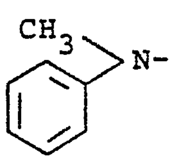 | $-S-CH_2-C_6H_5$ | 93-95 |

| Bsp. Nr. | A | B | Schmelzpunkt (°C) |
|---|---|---|---|
| 11 | 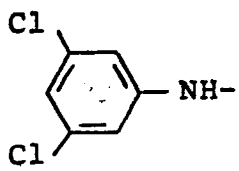 | $-S-CH_3$ | 140-144 |
| 12 | $CH_3O-$ | 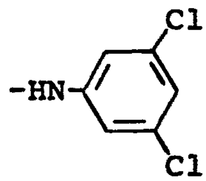 | 171-173 |
| 13 | 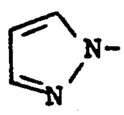 | $-OCH_3$ | 117-120 |
| 14 | $CH_3O-$ | 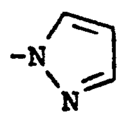 | Öl |
| 15 | 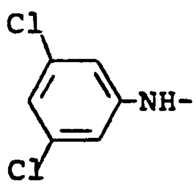 | 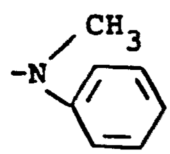 | 60-80 |
| 16 | $CH_3NH-$ | $-S-C_2H_5$ | 55-56 |
| 17 | 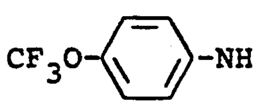 | $-OCH_3$ | 92-94 |

| Bsp. A<br>Nr. | B | Schmelzpunkt<br>(°C) |
|---|---|---|
| 18 | 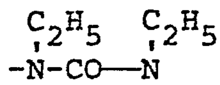 | 74-76 |
| 19 | 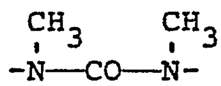 | Harz |
| 20 | 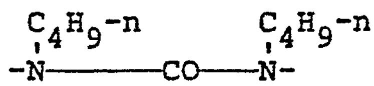 | Harz |

Anwendungsbeispiele

In den folgenden Anwendungsbeispielen wurden die nachstehend aufgeführten Verbindungen als Vergleichssubstanzen eingesetzt:

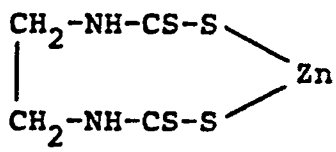

(A)

Zink-ethylen-1,2-bis-(dithiocarbamat),

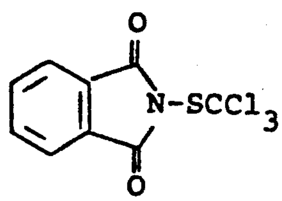

(B)

N-Trichlormethylthio-phthalimid

und

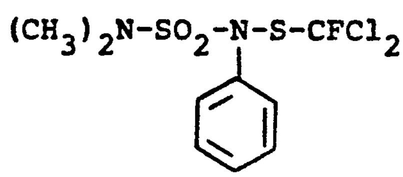

(C)

N-Dichlorfluormethylthio-N-phenyl-N',N'-dimethyl-sulfamid

0189800

Beispiel A

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 22°C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigt in diesem Test z.B. die Verbindung gemäß dem Herstellungsbeispiel: 1.

Beispiel B

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 1, 11 und 17.

Beispiel C

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 1, 3, 4, 8, 10, 20 und 2.

Beispiel D

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen bei diesem Test z.B. die Verbindungen gemäß den Herstellungsbeispielen: 18 und 19.

Beispiel E

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 6, 19, 20, 2 und 15.

## Patentansprüche

1. Thiocyanatomethylthio-Gruppen enthaltende Verbindungen der allgemeinen Formel (I)

$$A\text{-}CO\text{-}N{=}\overset{B}{\overset{|}{C}}\text{-}S\text{-}CH_2\text{-}SCN \qquad (I)$$

in welcher

A für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^1X$ steht, wobei

X für Sauerstoff, Schwefel oder $-NR^3$ steht,

$R^1$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für

gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl oder für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, außerdem für Wasserstoff stehen kann, wenn X für $-NR^3$ steht,

$R^3$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht,

B für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^2$-Y steht, wobei

Y für Sauerstoff, Schwefel oder $-NR^4$ steht,

$R^2$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl und für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, außerdem für Wasserstoff stehen kann, wenn Y für $-NR^4$ steht,

$R^4$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht, oder

A und B gemeinsam die Gruppe $-NR^5-CZ-NR^6-$ bedeuten, wobei

$R^5$ und $R^6$ unabhängig voneinander für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl oder für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, stehen und

Z für Sauerstoff oder Schwefel steht.

2. Thiocyanatomethylthio-Gruppen enthaltende Verbindungen der Formel (I) gemäß Anspruch 1, wobei

A für gesättigte oder ungesättigte 5- oder 6-gliedrige Heterocyclen mit 1 bis 4 gleichen oder verschiedenen Sauerstoff-, Stickstoff- oder Schwefelatomen stehen, die gegebenenfalls ein- bis fünffach, gleich oder verschieden durch Fluor, Chlor, Brom, Iod, Alkyl mit 1 bis 4 Kohlenstoffatomen und Halogenalkyl mit 1 bis 3 Kohlenstoffatomen und 1 bis 5 gleichen oder verschiedenen Fluor-, Chlor-, Brom-, Iodatomen substituiert sind und gegebenenfalls einen ankondensierten Benzolring enthalten, steht, oder für den Rest $R^1X$ steht, wobei

X für Sauerstoff, Schwefel oder $-NR^3$ steht,

$R^1$ für gegebenenfalls ein- bis fünffach, gleich oder verschieden durch Fluor-, Chlor-, Brom- und Iodatome, durch Nitro, Rhodan, Cyan, Alkoxy mit 1 bis 4 Kohlenstoffatomen und Alkylthio mit 1 bis 4 Kohlenstoffatomen substituiertes Alkyl mit 1 bis 16 Kohlenstoffatomen, für Alkenyl oder Alkinyl mit jeweils 2 bis 6 Kohlenstoffatomen, für Halogenalkenyl oder Halogenalkinyl mit jeweils 2 bis 6 Kohlenstoffatomen und 1 bis 5 gleichen oder verschiedenen Fluor-, Chlor-, Brom- und Iodatomen,

für jeweils gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Halogenalkyl mit 1 bis 4 Kohlenstoffatomen und 1 bis 5 gleichen oder verschiedenen Fluor-, Chlor-, Brom- und Iodatomen, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Fluor-, Chlor-, Brom- und Iodatomen, jeweils substituiertes Cycloalkyl oder Cycloalkenyl mit 5 bis 7 Kohlenstoffatomen, für gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Halogenalkyl mit 1 bis 4 Kohlenstoffatomen und 1 bis 5 gleichen oder verschiedenen Fluor-, Chlor-, Brom- und Iodatomen, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Fluor-, Chlor-, Brom- und Iodatomen, jeweils im Arylteil substituiertes Aralkyl oder Aryl mit jeweils 6 bis 10 Kohlenstoffatomen im Aryl und gegebenenfalls 1 bis 4 Kohlenstoffatomen im Alkylteil, und für 5- oder 6-gliedrige Heterocyclen mit 1 bis 4 gleichen oder verschiedenen Heteroatomen, wie Sauerstoff,

Schwefel und Stickstoff, die gleich oder verschieden, ein- bis vierfach durch Fluor, Chlor, Brom, Iod, Alkyl mit 1 bis 4 Kohlenstoffatomen und Halogenalkyl mit 1 bis 4 Kohlenstoffatomen und 1 bis 5 gleichen oder verschiedenen Fluor-, Chlor-, Brom- und Iodatomen substituiert sein können und einen ankondensierten Benzolring enthalten können, steht, außerdem für Wasserstoff stehen kann, wenn X für $-NR^3$ steht,

$R^3$ für Wasserstoff, für Alkyl mit 1 bis 6 Kohlenstoffatomen, für gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und jeweils 1 bis 5 Fluor-, Chlor-, Brom- und Iodatomen, jeweils substituiertes Aralkyl und Aryl mit 6 bis 10 Kohlenstoffatomen je Aryl und 1 bis 4 Kohlenstoffatomen im Alkylteil steht,

B für gesättigte oder ungesättigte 5- oder 6-gliedrige Heterocyclen mit 1 bis 4 gleichen oder verschiedenen Sauerstoff-, Stickstoff- oder Schwefelatomen steht, die gegebenenfalls ein- bis fünffach, gleich oder verschieden durch Fluor, Chlor, Brom, Iod, Alkyl mit

1 bis 4 Kohlenstoffatomen und Halogenalkyl mit 1 bis 3 Kohlenstoffatomen und 1 bis 5 gleichen oder verschiedenen Fluor-, Chlor-, Brom- und Iodatomen substituiert sind und gegebenenfalls einen ankondensierten Benzolring enthalten, steht oder für den Rest $R^2Y$ steht, wobei

Y für Sauerstoff, Schwefel oder $-NR^4$ steht,

$R^2$ für gegebenenfalls ein- bis fünffach, gleich oder verschieden durch Fluor, Chlor, Brom, Iod, Nitro, Rhodan, Cyan, Alkoxy mit 1 bis 4 Kohlenstoffatomen und Alkylthio mit 1 bis 4 Kohlenstoffatomen substituiertes Alkyl mit 1 bis 16 Kohlenstoffatomen, für Alkenyl mit 2 bis 6 Kohlenstoffatomen, für Halogenalkenyl mit 2 bis 6 Kohlenstoffatomen und 1 bis 5 gleichen oder verschiedenen Fluor-, Chlor-, Brom-, Iodatomen, für jeweils gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Halogenalkyl mit 1 bis 4 Kohlenstoffatomen und 1 bis 5 gleichen oder verschiedenen Fluor-, Chlor-, Brom-, Iodatomen, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Fluor-, Chlor-, Brom- und Iodatomen, jeweils substituiertes Cycloalkyl oder Cycloalkenyl mit 5 bis 7 Kohlenstoffatomen, für gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Halogenalkyl mit 1 bis 4 Kohlenstoffatomen und 1 bis 5 gleichen oder verschiedenen Fluor-, Chlor-, Brom- und Iodatomen, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Fluor-, Chlor-, Brom- und Iodatomen jeweils im Arylteil substituiertes Aralkyl oder Aryl mit jeweils 6 bis 10 Kohlenstoffatomen im Aryl und gegebenenfalls 1 bis 4 Kohlenstoffatomen im Alkylteil, und für 5- oder 6-gliedrige Heterocyclen mit 1 bis 4 gleichen oder verschiedenen Sauerstoff-, Schwefel- oder Stickstoffatomen, die gleich oder verschieden, ein- bis vierfach durch Fluor-, Chlor-, Brom- und Iodatomen, Alkyl mit 1 bis 4 Kohlenstoffatomen und Halogenalkyl mit 1 bis 4 Kohlenstoffatomen und 1 bis 5 gleichen oder verschiedenen Fluor-, Chlor-, Brom- und Iodatomen substituiert sein können und einen ankondensierten Benzolring enthalten können, außerdem für Wasserstoff stehen kann, wenn Y für $-NR^4$ steht,

$R^4$ für Wasserstoff, für Alkyl mit 1 bis 6 Kohlenstoffatomen, für gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Alkoxy oder Alkylthio mit jeweils 1 bis 4 Kohlenstoffatomen, Halogenalkyl, Halogenalkoxy oder Halogenalkylthio mit jeweils 1 bis 4 Kohlenstoffatomen und jeweils 1 bis 5 Fluor-, Chlor-, Brom- und Iodatomen jeweils substituiertes Aralkyl und Aryl mit 6 bis 10 Kohlenstoffatomen je Aryl und 1 bis 4 Kohlenstoffatomen im Alkylteil steht, oder

A und B gemeinsam die Gruppe $-NR^5-CZ-NR^6-$ bedeuten, wobei

$R^5$ und $R^6$ unabhängig voneinander für gegebenenfalls ein- bis fünffach, gleich oder verschieden durch Fluor-, Chlor-, Brom- und Iodatome, Nitro, Rhodan, Cyan, Alkoxy oder Alkylthio mit jeweils 1 bis 4 Kohlenstoffatomen substituiertes Alkyl mit 1 bis 6 Kohlenstoffatomen, für Alkenyl oder Alkinyl mit jeweils bis zu 6 Kohlenstoffatomen, für Halogenalkenyl oder Halogenalkinyl mit bis zu 4 Kohlenstoffatomen und 1 bis 5 gleichen oder verschiedenen Fluor, Chlor, Brom und Iod, für jeweils gegebenenfalls ein- bis fünffach, gleich oder verschieden durch Alkyl mit 1 bis 4 Kohlenstoffatomen, Fluor, Chlor,

Brom, Iod, Nitro, Rhodan, Cyan, Halogenalkyl mit 1 bis 4 Kohlenstoffatomen und 1 bis 5 gleichen oder verschiedenen Fluor-, Chlor-, Brom- und Iodatomen, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Fluor-, Chlor-, Brom- und Iodatomen, jeweils substituiertes Cycloalkyl oder Cycloalkenyl mit 5 bis 7 Kohlenstoffatomen, für gegebenenfalls ein- bis fünffach, gleich oder verschieden durch Alkyl mit 1 bis 4 Kohlenstoffatomen, Fluor, Chlor, Brom, Iod, Nitro, Rhodan, Cyan, Halogenalkyl mit 1 bis 4 Kohlenstoffatomen und 1 bis 5 gleichen oder verschiedenen Fluor-, Chlor-, Brom- und Iodatomen, Alkoxy oder Alkylthio mit jeweils 1 bis 4 Kohlenstoffatomen je Alkylteil, Halogenalkoxy oder Halogenalkylthio mit jeweils 1 bis 4 Kohlenstoffatomen und 1 bis 5 gleichen oder verschiedenen Fluor-, Chlor-, Brom- und Iodatomen, jeweils im Arylteil substituiertes Aralkyl oder Aryl mit jeweils 6 bis 10 Kohlenstoffatomen im Aryl und gegebenenfalls 1 bis 4 Kohlenstoffatomen im Alkylteil, und für 5- oder 6-gliedrige Heterocyclen mit 1 bis 4 gleichen oder verschiedenen Sauerstoff-, Schwefel- oder Stickstoffatomen, die gleich oder verschieden ein- bis vierfach durch Fluor, Chlor, Brom, Iod,

Alkyl mit 1 bis 4 Kohlenstoffatomen und Halogenalkyl mit 1 bis 4 Kohlenstoffatomen und 1 bis 5 gleichen oder verschiedenen Fluor-, Chlor-, Brom- und Iodatome substituiert sein können und einen ankondensierten Benzolring enthalten können, stehen und

Z für Sauerstoff oder Schwefel steht.

3. Thiocyanatomethylthio-Gruppen enthaltende Verbindungen der Formel (I) gemäß Anspruch 1, wobei

A für Piperidin, Piperazin, Pyrrolidin, Imidazolidin, Imidazol, Benzimidazolin, Pyrazolin, Carbazolin, Morpholin, Thiomorpholin, für $R^1X$ steht, wobei

X für Sauerstoff oder $-NR^3$ steht,

$R^1$ für gegebenenfalls ein- bis fünffach, gleich oder verschieden durch Fluor und Chlor, Methoxy, Ethoxy, Methylthio oder Ethylthio substituiertes Alkyl mit 1 bis 6 Kohlenstoffatomen steht, außerdem für Wasserstoff steht, wenn X für $-NR^3$ steht,

$R^3$ für Wasserstoff, für Alkyl mit 1 bis 8 Kohlenstoffatomen, für gegebenenfalls ein- bis dreifach, gleich oder verschieden durch Fluor, Chlor, Methyl, Ethyl, n- und iso-Propyl, Trifluormethyl, Trichlormethyl, Trifluormethoxy,

Trichlormethoxy, Trifluormethylthio, Dichlorfluormethyl, Dichlorfluormethoxy, jeweils substituiertes Phenyl, Benzyl oder Phenethyl steht,

B für Piperidin, Piperazin, Pyrrolidin, Imidazolin, Imidazolidin, Benzimidazolin, Pyrazolin, Carbazolin, Morpholin oder Thiomorpholin steht, weiterhin für den Rest $R^2Y$ steht, wobei

Y für Sauerstoff, Schwefel oder $-NR^4$ steht,

$R^2$ für gegebenenfalls ein- bis vierfach durch Fluor, Chlor, Methoxy, Ethoxy, Methylthio und Ethylthio substituiertes Alkyl mit 1 bis 14 Kohlenstoffatomen, für Benzyl, für gegebenenfalls ein- bis dreifach, gleich oder verschieden durch Fluor, Chlor, Methyl, Ethyl, Methoxy, Ethoxy, Methylthio, Ethylthio, Trifluormethyl, Trichlormethyl, Dichlorfluormethyl, Dichlorfluormethoxy oder Tetrachlorethyl substituiertes Phenyl steht, außerdem für Wasserstoff stehen kann, wenn Y für $-NR^4$ steht,

$R^4$ für Wasserstoff, für Alkyl mit 1 bis 4 Kohlenstoffatomen oder für gegebenenfalls ein- bis vierfach durch Fluor, Chlor, Methoxy, Ethoxy, Methylthio und Ethylthio substituiertes Alkyl mit 1 bis 14 Kohlenstoffatomen, für Benzyl, für gegebenenfalls ein- bis dreifach, gleich oder

verschieden durch Fluor, Chlor, Methyl, Ethyl, Methoxy, Ethoxy, Methylthio, Ethylthio, Trifluormethyl, Trichlormethyl, Dichlorfluormethyl, Dichlorfluormethoxy oder Tetrachlorethyl substituiertes Phenyl steht, oder

A und B gemeinsam für die Gruppe $-NR^5-CZ-NR^6-$ stehen, wobei

$R^5$ und $R^6$ unabhängig voneinander für gegebenenfalls durch Fluor, Chlor, Methoxy, Ethoxy, Methylthio oder Ethylthio substituiertes Alkyl mit 1 bis 4 Kohlenstoffatomen oder für Phenyl stehen und

Z für Sauerstoff steht.

4. Verfahren zur Herstellung von Thiocyanatomethylthio-Gruppen enthaltenden Verbindungen der Formel (I)

$$A-CO-N=\overset{\displaystyle B}{\overset{|}{C}}-S-CH_2-SCN \qquad (I)$$

in welcher

A für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^1X$ steht, wobei

X für Sauerstoff, Schwefel oder $-NR^3$ steht,

$R^1$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkyl und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl und für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, außerdem für Wasserstoff stehen kann, wenn X für $-NR^3$ steht,

$R^3$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht,

B für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, steht, oder für den Rest $R^2Y$ steht, wobei

Y für Sauerstoff, Schwefel oder $-NR^4$ steht,

$R^2$ für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl und für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, außerdem für Wasserstoff stehen kann, wenn Y für $-NR^4$ steht,

$R^4$ für Wasserstoff, für Alkyl, für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Aralkyl oder Aryl steht, oder

A und B gemeinsam die Gruppe $-NR^5-CZ-NR^6-$ bedeuten, wobei

$R^5$ und $R^6$ unabhängig voneinander für gegebenenfalls ein- bis mehrfach, gleich oder verschieden durch Halogen, Nitro, Rhodan, Cyan, Alkoxy und Alkylthio substituiertes Alkyl, für Alkenyl, für Halogenalkenyl, für Alkinyl, für Halogenalkinyl für gegebenenfalls ein- bis mehrfach, gleich oder verschieden durch Alkyl, Halogen, Nitro, Rhodan, Cyan, Halogenalkyl, Alkoxy, Alkylthio, Halogenalkoxy und Halogenalkylthio jeweils substituiertes Cycloalkyl oder Cycloalkenyl, für gegebenenfalls ein- bis mehrfach, gleich oder verschieden durch Alkyl, Halogen,

0189800

Nitro, Rhodan, Cyan, Halogenalkyl, Alkoxy, Alkylthio, Halogenalkoxy und Halogenalkylthio jeweils substituiertes Aralkyl oder Aryl und für 5- oder 6-gliedrige Heterocyclen, die 1 bis 4 gleiche oder verschiedene Heteroatome enthalten und gegebenenfalls ein- bis mehrfach, gleich oder verschieden durch Halogen, Alkyl und Halogenalkyl substituiert sind und gegebenenfalls ankondensierte Ringe enthalten, stehen und

Z für Sauerstoff oder Schwefel steht,

dadurch gekennzeichnet, daß man Verbindungen der allgemeinen Formel (II)

A-CO-NH-CS-B (II)

in welcher

A und B die oben angegebene Bedeutung haben

mit Halogenmethylthiocyanaten der Formel (III)

$Hal-CH_2-SCN$ (III)

in welcher

Hal für Chlor, Brom oder Iod steht,

in Gegenwart von Verdünnungsmitteln und gegebenenfalls in Gegenwart einer Base bei Temperaturen zwischen -50°C und +120°C umsetzt.

5. Schädlingsbekämpfungsmittel, gekennzeichnet durch einen Gehalt an mindestens einer Thiocyanatomethylthio-Gruppen enthaltende Verbindung der Formel (I) gemäß den Ansprüchen 1 und 4.

6. Verwendung von Thiocyanatomethylthio-Gruppen enthaltende Verbindungen der Formel (I) gemäß den Ansprüchen 1 und 4 zur Bekämpfung von Schädlingen.

7. Verfahren zur Bekämpfung von Schädlingen, dadurch gekennzeichnet, daß man Thiocyanatomethylthio-Gruppen enthaltende Verbindungen der Formel (I) gemäß den Ansprüchen 1 und 4 auf Schädlinge und/oder ihren Lebensraum einwirken läßt.

8. Verfahren zur Herstellung von Schädlingsbekämpfungsmitteln, dadurch gekennzeichnet, daß man Thiocyanatomethylthio-Gruppen enthaltende Verbindungen der Formel (I) gemäß den Ansprüchen 1 und 4 mit Streckmitteln und/oder oberflächenaktiven Mitteln vermischt.

9. Verwendung von Thiocyanatomethylthio-Gruppen enthaltende Verbindungen der Formel (I) gemäß Anspruch 6 zur Bekämpfung von Pilzen.

10. Verfahren gemäß Anspruch 7, dadurch gekennzeichnet, daß man Thiocyanatomethylthio-Gruppen enthaltende Verbindungen der Formel (I) auf Pilze und/oder ihren Lebensraum einwirken läßt.